# EUROPÄISCHE PATENTANMELDUNG

(11) **EP 0 619 310 A1**
(43) Veröffentlichungstag der Anmeldung: **12.10.1994**
(21) Anmeldenummer: 94105208.6
(22) Anmeldetag: 01.04.1994
(51) Int. Cl.: C07D 239/545

(54) **Verbessertes Verfahren zur Herstellung von 1,3-Dimethyl-4,5-diaminouracil**

(30) Priorität: 07.04.1993 DE 4311538
(71) Anmelder: BOEHRINGER INGELHEIM KG, D-55216 Ingelheim (DE); BOEHRINGER INGELHEIM INTERNATIONAL GmbH, D-55216 Ingelheim am Rhein (DE)
(72) Erfinder: Dach, Rolf, Dr. Dipl.-Chem., D-55435 Gau-Algesheim (DE); Goldschmidt, Wilfried, D-55218 Ingelheim/Rhein (DE)

(57) **Zusammenfassung**

Die vorliegende Erfindung betrifft ein verbessertes Vefahren zur Herstellung von 1,3-Dimethyl-4,5-diamino-uracil durch katalytische Reduktion von 1,3-Dimethyl-4-amino-5-nitrosouracil in Gegenwart eines Palladium-Kohle-Katalysators.

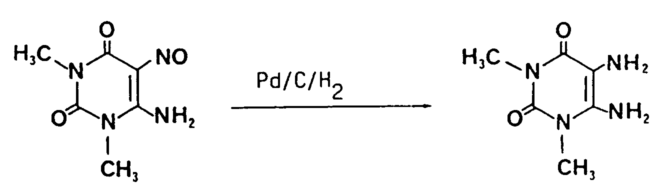

## Beschreibung

Die vorliegende Erfindung betrifft ein verbessertes Verfahren zur Herstellung von 1,3-Dimethyl-4,5-diaminouracil (I) 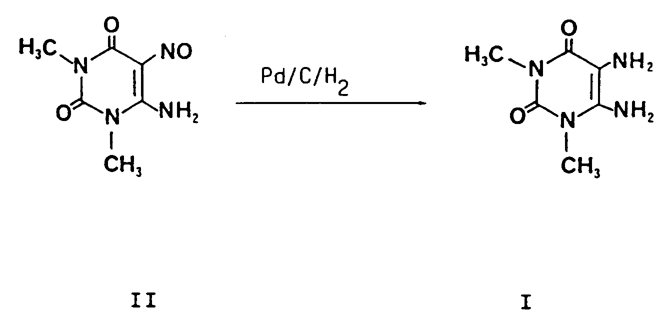
Der Grundkörper des 4,5-Diaminouracils (I) verkörpert einen wichtigen Synthesebaustein in Synthesen, welche die Herstellung von solchen Purinderivaten betreffen - wie z. B. Theophyllin, Coffein oder Theobromin.

Von diesen Purinderivaten besitzen einige wertvolle pharmakologische Eigenschaften. So ist beispielsweise vom Theophyllin bekannt, daß es gegen Bronchialasthma wirkt und die Leistung des Herzens sowie die Durchblutung der Koronargefäße steigert. Theophyllin gehört somit in die Gruppe der sehr wichtigen Herzmittel und wirkt daneben als Diuretikum.

Der Ausgangsbaustein für die Synthese des Theophyllins wird vom 1,3-Dimethyl-4,5-diaminouracil (I) verkörpert, das beispielsweise mit Ameisensäure leicht in Theophyllin überführt werden kann.

Im Hinblick auf den großen Bedarf an diesem Purinabkömmling und dessen Verwendung als pharmazeutischer Wirkstoff besteht ein großes Interesse, dieses Produkt in einer guten Ausbeute und in guter Qualität, d. h. in erster Linie in hoher Reinheit zur Verfügung zu stellen.

Verfahren zur Reduktion des 1,3-Dimethyl-4-amino-5-nitrosouracils (II) zum 1,3-Dimethyl-4,5-diaminouracil sind aus dem Stand der Technik bekannt. So ist zum Beispiel die Reduktion des Nitrosouracilderivats II mittels Schwefelwasserstoff in flüssigem Ammoniak bekannt [H. Krahnefeld, J. Wolf, S. Stutzriemer, H. Zumpe, K.D. Fritsche und G. Hockeborn, DD 212,253].

Daneben ist die entsprechende Reduktion auf elektrolytischem Wege bekannt [A. N. Dolgachev, Avrutskaja and Y. M. Fioshin, Elektrokhimiya 15(12) (1979) 1882].

Weiterhin ist die katalytische Reduktion des 1,3-Dimethyl-4-amino-5-nitrosouracils (II) zum 1,3-Dimethyl-4,5-diaminouracil (I) unter Verwendung von Palladiumkatalysatoren bekannt [N.A. Frolova, Z. A. Yugai, K. M. Makhkamov und E. A. Sapozhnikova, Khim.-Farm. Zh. 12(1) (1988) 67].

Alle angeführten Synthesen eignen sich nicht oder nur unter großen Einschränkungen zur Herstellung des Diaminouracilderivats in industriellen Herstellverfahren.

So berichten N. A. Frolova et al., daß die Verwendung eines Palladium-Kohle-Katalysators für großtechnische Zwecke mit einer Inkonstanz bezüglich der Produktausbeute sowie im Hinblick auf die Produktqualität verbunden ist und daß die während der Reaktion eintretende Schaumbildung verfahrenstechnische Schwierigkeiten bereitet.

Überraschenderweise wurde nun gefunden, daß die Verwendung eines Palladium-Kohle-Katalysators bei Einhaltung bestimmter Reaktionsbedingungen das 1,3-Dimethyl-4,5-diaminouracil (I) durchweg in quantitativer Ausbeute und in hoher Reinheit verfügbar macht. Dabei sind Qualitätsmerkmale des Reduktionsproduktes so gut, daß dadurch eine direkte Weiterverarbeitung des Reduktionsproduktes zum Theophyllin bzw. dessen Caliumsalz - ohne Isolierung und weitere Reinigung des 1,3-Dimethyl-4,5-diamino-uracils (I) oder dessen sogenannten Formylkörpers - ermöglicht wird. Ein weiterer Vorzug des erfindungsgemäßen Verfahrens besteht darin, daß die Hydrierung in Suspensionen erfolgen kann, womit eine Hydrierung bei einem niedrigen Temperaturniveau ermöglicht wird.

Dazu wird eine wässerige Suspension des 1,3-Dimethyl-4-amino-5-nitrosouracils (II) mit einem Palladium-Kohle-Katalysator - bevorzugt mit einem Palladiumgehalt von 5 % - versetzt. In der so erhaltenen Suspension wird zu Beginn der Reaktion der pH-Wert mit der wässerigen Lösung einer alkalisch reagierenden Verbindung - bevorzugt mit der wässerigen Lösung eines Alkalihydroxids und besonders bevorzugt mit konzentrierter Natronlauge - auf einen Wert im Bereich von 7 bis 10, bevorzugt 8 bis 9,5 und besonders bevorzugt wird pH 9 - eingestellt.

Die so behandelte Suspension wird anschließend unter einem Wasserstoffdruck im Bereich von 1 bis 20 bar - bevorzugt 2 bis 6 bar und besonders bevorzugt unter einem Wasserstoffdruck von 3 bar bei einer Temperatur im Bereich von 20 bis 60°C und bevorzugt im Bereich von 30 bis 50°C hydriert. Nach beendeter Wasserstoffaufnahme wird die Suspension auf eine Temperatur im Bereich von 15 bis 40°C - bevorzugt 20 bis 30°C - und mit Ameisensäure versetzt bis sämtliches in 1,3-Dimethyl-4,5-diaminouracil (I) als Formiat in Lösung gegangen ist. Von der so erhaltenen Reaktionslösung kann der Katalysator nunmehr zweckmäßigerweise auf dem Wege der Filtration abgetrennt werden. Die HPLC-Analyse des Filtrates belegt, daß sich das 1,3-Dimethyl-4,5-diaminouracil (I) zu 99,9 % - bezogen auf das eingesetzte 1,3-Dimethyl-4-amino-5-nitrosouracil (II) umgesetzt hat. Zu dem auf diesem Wege gewonnenen Filtrat fügt man noch einmal Ameisensäure zu und läßt das Reaktionsgemisch über einen Zeitraum von ca. 40 Minuten bei einer Temperatur von ca. 85°C ausreagieren und fällt anschließend das Calciumsalz des Theophyllins mit Calciumhydroxid-Suspension. Das Theophyllin-Calcium wird abfiltriert, mit Wasser gewaschen und bei einer Temperatur von ca. 60° C getrocknet.

Die beschriebene Erfindung wird nunmehr durch das in den Beispielen beschriebene Verfahren erläutert. Verschiedenartige, andere Ausgestaltungen des Verfahrens werden für den Fachmann aus der vorliegenden Beschreibung ersichtlich. Es wird jedoch ausdrücklich darauf hingewiesen, daß das Beispiel und die Beschreibung lediglich zur Erläuterung vorgesehen und nicht als Einschränkung der Erfindung anzusehen sind.

### Beispiel 1

254 g (81 nmol) 1,3-Dimethyl-4-amino-5-nitrosouracil (41 % Wassergehalt) wurden mit 310 ml Wasser und 17 g 5 %igem Palladium/Kohle-Katalysator (alkalisch gefällt) versetzt. Anschließend wird mit konzentrierter Natronlauge in der Reaktionsmischung ein pH-Wert von 9 eingestellt. Die so erhaltene Suspension wird unter einem Wasserstoffdruck von 3 bar und unter Rühren innerhalb eines Temperaturintervalls von 30 auf 50° C hydriert. Nach beendeter Wasserstoffaufnahme wird die Reaktionsmischung auf eine Temperatur im Bereich von 20 bis 30°C abgekühlt und mit 40 g (869 mmol) Ameisensäure versetzt und anschließend vom Katalysator abfiltriert. Zu dem Filtrat fügt man weitere 25 g (543 mmol) Ameisensäure zu und läßt über einen Zeitraum von ca. 40 Minuten bei einer Temperatur von 85° C ausreagieren. Zu dem so erhaltenen Gemisch fügt man 65 g Calciumhydroxid-Suspension, worauf das Caliumsalz des Theophyllins ausfällt. Das ausgefallene Calciumsalz wird abfiltriert, mit 200 ml Wasser gewaschen und das so isolierte Calciumsalz wird im Umlufttrockenschrank ca. 12 Stunden bei 60° C getrocknet. Auf diesem Wege werden 79 % an Theophyllincalcium bezogen auf das eingesetzte 1,3-Dimethyl-4-amino-5-nitrosouracil gewonnen.

## Patentansprüche

1. Verfahren zur Herstellung von 1,3-Dimethyl-4,5-diaminouracil durch katalytische Reduktion von 1,3-Dimethyl-4-amino-5-nitrosouracil in Gegenwart eines Palladium-Kohle-Katalysators, dadurch gekennzeichnet, daß man in einer wässerigen Suspension das 1,3-Dimethyl-4-amino-5-nitrosouracil mit der wässerigen Lösung einer alkalisch reagierenden Verbindung einen pH-Wert im Bereich von 7 bis 10 einstellt und anschließend unter einem Wasserstoffdruck im Bereich von 1 bis 20 bar innerhalb eines Temperaturintervalls von 20 bis 60°C hydriert und die Reaktionsmischung nach beendeter Wasserstoffaufnahme bei einer Temperatur im Bereich von 15 bis 40°C mit Ameisensäure versetzt bis sämtliches 1,3-Dimethyl-4,5-diamino-uracil in Lösung gegangen ist, das Reaktionsgemisch vom Katalysator abfiltriert, und das 1,3-Dimethyl-4,5-diamino-uracil in Form seines Formiates isoliert oder weiter zu Theophyllin-Calcium umsetzt.

2. Verfahren nach Anspruch 1, dadurch gekennzeichnet, daß man in einer wässerigen Suspension des 1,3-Dimethyl-4-amino-5-nitrosouracil mit der wässerigen Lösung eines Alkalihydroxids einen pH-Wert im Bereich von 8 bis 9,5 einstellt und anschließend unter einem Wasserstoffdruck im Bereich von 2 bis 6 bar innerhalb eines Temperaturintervalls von 30 bis 50°C hydriert und die Reaktionsmischung nach beendeter Wasserstoffaufnahme bei einer Temperatur im Bereich von 20 bis 30°C mit Ameisensäure versetzt bis sämtliches 1,3-Dimethyl-4,5-diaminouracil in Lösung gegangen ist, das Reaktionsgemisch vom Katalysator abfiltriert und das 1,3-Dimethyl-4,5-diaminouracil in Form seines Formiates isoliert oder weiter zu Theophyllin-Calcium umsetzt.

3. Verfahren nach Anspruch 1, dadurch gekennzeichnet, daß man in einer wässerigen Suspension des 1,3-Dimethyl-4-amino-5-nitrosouracil mit Natronlauge einen pH-Wert von 9 einstellt und anschließend unter einem Wasserstoffdruck von 3 bar innerhalb eines Temperaturintervalls von 30 bis 50°C hydriert und die Reaktionsmischung nach beendeter Wasserstoffaufnahme bei einer Temperatur im Bereich von 20 bis 30 °C mit Ameisensäure versetzt bis sämtliches 1,3-Dimethyl-4,5-diaminouracil in Lösung gegangen ist, das Reaktionsemisch vom Katalysator abfiltriert, und das 1,3-Dimethyl-4,5-diaminouracil in Form seines Formiates isoliert oder weiter zu Theophyllin-Calcium umsetzt.
